# EUROPÄISCHE PATENTSCHRIFT

(11) **EP 3 021 877 B1**
(45) Veröffentlichungstag und Bekanntmachung des Hinweises auf die Patenterteilung: **27.03.2019**
(21) Anmeldenummer: 14741224.1
(22) Anmeldetag: 03.07.2014
(51) Int. Cl.: A61L 15/26, A61L 15/40, A61L 15/42

(54) **WUNDAUFLAGE**
WOUND DRESSING
PANSEMENT

(30) Priorität: 15.07.2013 DE 102013107464
(43) Veröffentlichungstag der Anmeldung: 25.05.2016
(73) Patentinhaber: Biowim Products GmbH, 79199 Kirchzarten (DE)
(72) Erfinder: FLEISCHMANN, Wilhelm, 79104 Freiburg (DE)
(74) Vertreter: Westphal, Mussgnug & Partner Patentanwälte mbB
(86) Internationale Anmeldenummer: PCT/EP2014/064267
(87) Internationale Veröffentlichungsnummer: WO 2015/007539

(56) Entgegenhaltungen:
- EP-A1- 1 020 197
- EP-A1- 2 014 314
- WO-A1-2006/054108
- DE-A1-102009 005 363

## Beschreibung

Die Erfindung betrifft eine Wundauflage in Form eines Fliegenmaden einschließenden Beutels mit einer porösen Wandung aus Kunststoff.

Zur Behandlung chronischer schlecht heilender Wunden ist es bekannt, lebende Fliegenmaden in die Wunde einzusetzen. Vorzugsweise werden hierzu die Maden der Schmeißfliege, z. B. Lucilia Sericata verwendet. Die Wirksamkeit der Maden beruht auf der Wundreinigung (Debridement), der antimikrobiellen Aktivität und der Stimulation der Wundheilung. Für diese Wirkungen sind im Wesentlichen die Verdauungsenzyme verantwortlich, die die Maden absondern.

Die Maden können als sogenannte Freiläufer in die offene Wunde eingesetzt werden, wobei sie durch ein aufgeklebtes Gaze-Netz an einem Entweichen aus der Wunde gehindert werden.

Aus WO2006/054108 A1 ist es bekannt, die Maden mittels eines Beutels in die Wunde einzubringen, der eine Wandung aus Kunststoff, z. B. Polyester, Polyethylen oder Polypropylen, aufweist. Die Wandung weist Öffnungen mit einem Durchmesser größer als 0,4 mm auf, sodass die Maden durch diese Poren hindurch als Freiläufer in die Wunde austreten können.

Bevorzugt wird heute eine Applikation der Maden mittels einer beutelförmigen Wundauflage, in welcher die Maden eingeschlossen sind. Die Wandung des Beutels ist porös, damit das von den Maden abgesonderte Sekret in die Wunde durchtreten kann und damit das durch das Madensekret aufgelöste nekrotische Gewebe in den Beutel eindringen und von den Maden aufgenommen werden kann. Eine solche Wundauflage ist aus der EP 1 020 197 B1 bekannt.

Bei dieser bekannten Wundauflage besteht die Wandung des Beutels aus einem feinmaschigen Netz aus Polyamid (z. B. NylonFasern) oder Polyestergarn mit Maschenweiten von etwa 0,12mm. Die Verwendung solcher Wundauflagen, die z. B. unter der Marke "BioBag" von der Firma Biomonde GmbH, Barsbüttel, Deutschland, auf den Markt gebracht werden, ist beispielsweise beschrieben in einem Artikel von G. Cazander et al "Maggot therapy for wound healing...", Journal of Wound Technology, July 2009, Seiten 18 bis 23. Um bei dieser bekannten Wundauflage ein Verkleben der Wandungen zu verhindern, wird in den Beutel vorzugsweise ein Abstandshalter, z. B. in Form eines PVA-Schwamms eingesetzt, wodurch den Maden ausreichend Raum freigehalten wird.

Bei dieser bekannten Wundauflage muss das textile Netz der Wandung sehr feinmaschig sein, damit die Maden die Maschen nicht aufweiten und entweichen können. Diese Feinmaschigkeit wirkt sich nachteilig auf die Porosität der Wandung und damit auf die Flüssigkeitsdurchlässigkeit der Wandung aus. Es wurde daher versucht, den Beutel aus dem feinmaschigen Polyamid-Netz mit einer Polyvinylalkohol-Feuchtmembran zu umschließen. Eine solche Wundauflage wurde von der oben genannten Biomonde GmbH unter der Bezeichnung "VitaPad" angeboten. Die PVA-Ummantelung verringert allerdings zusätzlich die Porosität. PVA-Schaumstoff findet als Hydroschwamm Anwendung als heilungsfördernde Wundauflage. Der Wassergehalt des PVA-Materials als Ummantelung kann allerdings wegen der starken Verdunstung ein Austrocknen der Maden nur kurzzeitig verhindern. Der gesamte Verband muss daher etwa 3 mal täglich befeuchtet werden, um die Maden am Leben zu halten. Außerdem muss die PVA-Membran nass gehalten werden, da sie bei Trocknung hart wird, sich nicht mehr an die Wunde anschmiegt und gegebenenfalls die Wundränder schädigt. Das häufige Befeuchten macht die Wundbehandlung zeitintensiv. Die Verdunstungskälte senkt die Temperatur des Verbandes und damit der Wunde, was das Madenwachstum und die Wundheilung nachteilig beeinflusst. Insbesondere begünstigt das ständige Nasshalten des Verbandes das Wachstum mikrobieller Krankheitserreger, sogenannter Nasskeime. Hierdurch verursachte Infektionen stellen eine gefährliche Komplikation der Wundbehandlung dar. Die Anwendung dieser Wundauflage ist daher problematisch, weshalb sie inzwischen vom Markt genommen wurde.

Aus EP2014314A1 ist es bekannt, einen offenzelligen Polyurethan-Schaumstoff als Wundauflage zu verwenden. Als Material für einen Beutel für die Madentherapie ist der Schaumstoff nicht vorgesehen.

Der Erfindung liegt die Aufgabe zu Grunde, eine Wundauflage zur Verfügung zu stellen, die bei einfacher Anwendung die Effektivität und Sicherheit der Therapie mit Fliegenmaden verbessert.

Diese Aufgabe wird erfindungsgemäß gelöst durch eine Wundauflage mit den Merkmalen des Patentanspruchs 1.

Vorteilhafte Ausführungen und Weiterbildungen der Erfindung sind in den Unteransprüchen angegeben.

Erfindungsgemäß wird die Wundeauflage in Form eines lebende Fliegenmaden einschließenden Beutels mit einer Wandung hergestellt, die aus einer Membran aus einem total offenporigen Polyurethan-Schaumstoff (PUR-Schaumstoff) besteht, dessen Porendurchmesser 0,1 mm bis 0,4 mm beträgt. Die offenporige Struktur der PUR-Membran wird durch Retikulieren erzeugt. Die vollständig durchgängig offenporige Struktur gewährleistet die Flüssigkeitsdurchlässigkeit der Wandung des Beutels. Der PUR-Schaumstoff wird nach dem Retikulieren verpresst, wodurch die Stabilität des Schaumstoffes erhöht und die Porengröße verringert werden.

Die Verwendung einer offenporigen PUR-Membran bietet überraschende wesentliche Vorteile gegenüber den bekannten Wundauflagen.

Klinische Anwendungen haben überraschend gezeigt, dass es möglich ist, auch den von der PUR-Trockenmembran umhüllten Maden optimale Umgebungsbedingungen zu gewährleisten, sofern die Membran der Wundauflage unmittelbar auf der Wundoberfläche aufliegt. Die verdunstende Oberfläche der PUR-Membran ist wesentlich geringer im Vergleich zur PVA-Feuchtmembran. Die Feuchtigkeit des Wundsekrets reicht daher meistens allein schon für das Überleben und Wachstum der Maden aus. Das pflegeintensive Anfeuchten und Nasshalten des Wundverbandes ist daher nur ausnahmsweise erforderlich. Die Bildung von Nasskeimen ist weitgehend vermieden.

Während bei den Wundauflagen mit einem feinmaschigen Polyamid-netz eine sehr geringe Maschenweite notwendig ist, um ein Aufweiten der Maschenöffnungen durch die Maden zu verhindern, lässt der PUR-Schaum eine Aufweitung der Porengröße durch die Maden praktisch nicht zu. Die Membran kann daher mit einem größeren Porendurchmesser und einer geringeren Dicke hergestellt werden, was die Durchlässigkeit der Membran für das Madensekret und das verflüssigte nekrotische Gewebe verbessert.

Ein weiterer überraschender Vorteil ergibt sich daraus, dass der PUR-Schaumstoff mit einer sehr einheitlichen Porengröße herstellbar ist. Es kann daher eine Membran verwendet werden, deren Porendurchmesser so groß gewählt wird, dass die Maden gerade noch nicht in die Poren eindringen können. Die Herstellungs- und Verarbeitungstechnik des PUR-Schaumstoffes ermöglicht es, die Streuungsbreite der Porengröße so weit zu reduzieren, dass das sichere Einschließen der Maden in den Beutel der Wundauflage mit einer optimalen Porosität der Membran vereinbar ist.

Ein weiterer wesentlicher Vorteil der PUR-Membran besteht darin, dass diese PUR-Membran weich und anschmiegsam ist, sodass sich die Wundauflage dicht an die Wundoberfläche anlegt, wodurch die Wirksamkeit der Maden und des Madensekrets begünstigt wird. Die weiche Biegsamkeit der PUR-Membran ist dabei von den Umgebungsbedingungen, d. h. insbesondere auch von der Feuchtigkeit unabhängig. Es ist daher nicht notwendig, die Feuchtigkeit der Wundauflage zu überwachen und zu beeinflussen. Dadurch wird die Anwendung der Wundauflage wesentlich vereinfacht.

Üblicherweise wird die beutelförmige Wundauflage aus zwei aufeinander gelegten Membranflächen hergestellt, die an den Rändern miteinander verbunden werden, z. B. durch Verkleben oder Verschweißen. Auch die Nähte, an welchen die Ränder miteinander verbunden sind, bleiben weich und biegsam, sodass sie das Anliegen der Wundauflage an die Wundoberfläche nicht beeinträchtigen und die Wundoberfläche nicht schädigen. Der niedrige Schmelzpunkt des PUR ermöglicht insbesondere ein Verschweißen mit einfachen, kostengünstigen Hilfsmitteln und hoher Zuverlässigkeit.

Die im Inneren des Beutels aneinander anliegenden Wandungen neigen auf Grund der Materialeigenschaften des PUR-Schaums nicht zum Verkleben. Die sich entwickelnden und wachsenden Maden können daher die weichen und biegsamen Wandungen des Beutels auseinander drücken und werden in ihrer Entwicklung nicht behindert, ohne dass zusätzlich ein Abstandshalter in den Beutel eingesetzt werden muss.

Die großporige dünne Membran des PUR-Schaumstoffs weist eine relativ geringe Oberfläche auf, sodass die Flüssigkeitsverdunstung gering ist. Es besteht daher eine geringere Gefahr, dass die Wundauflage austrocknet und die eingeschlossenen Maden vertrocknen.

Der Polyurethan-Schaum kann auf Polyether- oder Polyesterbasis hergestellt werden. Der PUR-Schaumstoff der Membran hat einen ähnlich heilungsfördernden Einfluss auf die Wunde, wie er von PVA-Wundauflagen bekannt ist. Ein PUR-Schaum auf Polyetherbasis ist zu bevorzugen wegen seiner sehr guten Hydrolysebeständigkeit, seiner Beständigkeit gegen Säuren und Basen, seiner sehr guten Kälteflexibilität und insbesondere auch seiner Mikrobenbeständigkeit.

Die Membran aus dem PUR-Schaumstoff kann mit einer Dicke, d. h. Wandstärke von 0,5 mm oder weniger hergestellt werden. Vorzugsweise kann die Dicke der Membran etwa 0,1 mm betragen. Diese geringe Dicke begünstigt die weiche Biegsamkeit der Membran und die gute Flüssigkeitsdurchlässigkeit.

Der Durchmesser der Poren der PUR-Membran wird möglichst groß gewählt, um auf der Wundseite eine möglichst gute Flüssigkeitsdurchlässigkeit und auf der Außenseite eine gute Belüftung der Maden zu gewährleisten. Die obere Grenze des Porendurchmessers ist durch die Größe und insbesondere durch den Durchmesser der Maden bestimmt und beträgt 0,4 mm Der Durchmesser der Poren wird etwas kleiner als der Durchmesser der Maden gewählt, sodass diese nicht in die Poren eindringen, diese aufdehnen und durch die Wandung entweichen können.

Bei der Schmeißfliege Lucilia Sericata weisen die Fliegeneier beispielsweise einen durchschnittlichen Durchmesser von 0,47 mm auf. Im ersten Larvenstadium weisen die frisch geschlüpften Maden einen durchschnittlichen Durchmesser von etwa 0,75 mm auf, wobei hier eine starke Schwankung von +/- 50% zu beobachten ist. Im zweiten Larvenstadium weisen die Maden einen durchschnittlichen Durchmesser von 1,37 mm auf, wobei hier nur noch kleinere Schwankungen von +/- 20% beobachtet werden.

Aus diesen Werten ergibt sich, dass der Porendurchmesser der PUR-Membran wenigstens 0,1 mm betragen soll. Ein kleinerer Porendurchmesser würde nur die Flüssigkeitsdurchlässigkeit verringern, hätte jedoch keinen Vorteil für das Einschließen der Maden. Eine obere Grenze des Porendurchmessers ergibt sich aus der Größe der jeweils verwendeten Maden. Für alle eventuell in Frage kommenden Fliegenmaden dürfte eine obere Grenze des Porendurchmessers von 1,0 mm nicht überschritten werden, er beträgt jedoch bis 0,4 mm. Für die heute in den meisten Fällen eingesetzten Maden der Lucilia Sericata wird entsprechend den oben angegebenen Maßen der Maden eine obere Grenze des Porendurchmessers von 0,4 mm verwendet. Um zuverlässig zu verhindern, dass die Maden mit ihrem etwas kegelförmig zulaufenden Kopfteil in die Poren eindringen, wird vorzugsweise ein Porendurchmesser von etwa 0,3 mm gewählt.

Um den sich entwickelnden Maden in dem Beutel der Wundauflage ausreichend Platz zu halten, kann in einer vorteilhaften Ausführung in dem Bereich der miteinander verbundenen Ränder der die Wandung des Beutels bildenden Membranflächen ein rahmenförmiger Abstandshalter eingesetzt sein. Dieser Abstandshalter vergrößert den lichten inneren Abstand der Wandungen, reduziert aber nicht die innere Flächenausdehnung des Beutels.

In einer Weiterbildung der Erfindung kann in den aus der PUR-Membran gebildeten Beutel ein zusätzlicher Innenbeutel eingelegt werden, dessen Wandung ebenfalls aus einer Membran aus einem offenporigen PUR-Schaumstoff besteht. Der Porendurchmesser der PUR-Membran des Innenbeutels ist ≤ 0,4 mm. Die Membran des Innenbeutels weist eine geringe Reißfestigkeit auf. Diese geringe Reißfestigkeit kann dadurch erzielt werden, dass die Membran des Innenbeutels sehr dünnwandig ist und/oder Sollbruchlinien aufweist. Solche Sollbruchlinien können beispielsweise die Schweiß- oder Klebnähte am Rand des Innenbeutels sein.

In dieser Ausführung werden die Eier der Fliege in den Innenbeutel eingelegt, der dann von dem äußeren Beutel umschlossen wird. Die Porengröße des Innenbeutels gewährleistet, dass die Fliegeneier nicht aus dem Innenbeutel herausrutschen können. Auch die aus den Eiern schlüpfenden winzigen Maden können auf Grund dieser Porengröße nicht aus dem Innenbeutel entweichen. Nach dem Schlüpfen nehmen die Maden in Kraft und Größe sehr schnell zu, sodass sie in dem Innenbeutel einen starken Druck erzeugen, der zu einem Reißen oder Platzen des Innenbeutels führt. Die Maden können sich dann frei in dem äußeren Beutel bewegen. Die Porengröße der PUR-Membran des äußeren Beutels kann in dieser Ausführung entsprechend der Größe der Maden gewählt werden, die im zweiten Larvenstadium aus dem Innenbeutel austreten. Die Porengröße der Wandung des äußeren Beutels weist einen Durchmesser von 0,1 mm bis 0,4 mm auf. Diese Ausführung hat den Vorteil, dass die Maden bereits als Eier in die Wundauflage eingebracht werden können und bereits im frühen ersten Larvenstadium aktiv zur Wundheilung beitragen können. Im zweiten Larvenstadium werden die Maden dann in dem äußeren Beutel gehalten, der einen wesentlich größeren Porendurchmesser und damit eine sehr hohe Durchlässigkeit aufweisen kann. Die sich im Laufe der Madenentwicklung ändernde Aktivität der Maden und die sich ändernde Zusammensetzung des Madensekrets können auf diese Weise über die ganze Entwicklungsdauer der Maden optimal ausgenutzt werden.

## Patentansprüche

1. Wundauflage in Form eines Fliegenmaden einschließenden Beutels mit einer porösen Wandung aus Kunststoff,
**dadurch gekennzeichnet, dass** die Wandung eine Membran aus einem total offenporigen Polyurethan-Schaumstoff aufweist, welcher retikuliert und verpresst ist und dessen Porendurchmesser 0,1 mm bis 0,4 mm beträgt.

2. Wundauflage nach Anspruch 1,
**dadurch gekennzeichnet, dass** der Porendurchmesser 0,3 mm beträgt

3. Wundauflage nach einem der Ansprüche 1 bis 2,
**dadurch gekennzeichnet, dass** die Membran eine Dicke ≤ 0,5 mm aufweist.

4. Wundauflage nach Anspruch 3,
**dadurch gekennzeichnet, dass** die Dicke 0,1 mm beträgt.

5. Wundauflage nach einem der vorhergehenden Ansprüche,
**dadurch gekennzeichnet, dass** der Polyurethan-Schaumstoff aus einem Polyurethan auf Polyether-basis besteht.

6. Wundauflage nach einem der vorhergehenden Ansprüche,
**dadurch gekennzeichnet, dass** der Beutel aus zwei aufeinander gelegten und am Rand miteinander verbundenen Membranflächen besteht.

7. Wundauflage nach Anspruch 6,
**dadurch gekennzeichnet, dass** im Bereich der miteinander verbundenen Ränder der Membranflächen ein rahmenförmiger Abstandshalter zwischen die Membranflächen eingesetzt ist.

8. Wundauflage nach einem der vorhergehenden Ansprüche,
**dadurch gekennzeichnet, dass** in dem Beutel ein Innenbeutel eingelegt ist, der aus einer Wandung aus einem offenporigen Polyurethan-Schaumstoff mit einem Porendurchmesser ≤ 0,4 mm und einer geringen Reißfestigkeit besteht.

## Claims

1. Wound dressing in the form of a bag enclosing fly maggots with a porous wall made from plastic, **characterised in that** the wall has a membrane made from a totally open-pored polyurethane foam, which is reticulated and compressed and of which the pore diameter is 0.1 mm to 0.4 mm.

2. Wound dressing according to claim 1, **characterised in that** the pore diameter is 0.3 mm.

3. Wound dressing according to one of claims 1 to 2, **characterised in that** the membrane has a thickness of ≤ 0.5 mm.

4. Wound dressing according to claim 3, **characterised in that** the thickness is 0.1 mm.

5. Wound dressing according to one of the preceding claims, **characterised in that** the polyurethane foam is made from a polyether-based polyurethane.

6. Wound dressing according to one of the preceding claims, **characterised in that** the bag consists of two membrane surfaces placed one upon the other and connected to one another at the edge.

7. Wound dressing according to claim 6, **characterised in that** a frame-shaped spacer is inserted between the membrane surfaces in the region of the membrane surfaces which are connected to one another.

8. Wound dressing according to one of the preceding claims, **characterised in that** an inner bag is inserted into the bag, wherein the inner bag consists of a wall made from an open-pored polyurethane foam with a pore diameter of ≤ 0.4 mm and a low tear resistance.

## Revendications

1. Pansement réalisé sous la forme d'un sac renfermant des asticots ayant une paroi poreuse en matériau synthétique,
**caractérisé en ce que**
la paroi comporte une membrane en mousse de polyuréthane à pores totalement ouverts qui est réticulée et comprimée et dont le diamètre des pores est égal à 0,1 mm à 0,4 mm.

2. Pansement conforme à la revendication 1,
**caractérisé en ce que**
le diamètre des pores est égal à 0,3 mm.

3. Pansement conforme à l'une des revendications 1 et 2,
**caractérisé en ce que**
la membrane a une épaisseur ≤ 0,5 mm.

4. Pansement conforme à la revendication 3,
**caractérisé en ce que**
l'épaisseur est égale à 0,1 mm.

5. Pansement conforme à l'une des revendications précédentes,
**caractérisé en ce que**
la mousse de polyuréthane est constituée par un polyuréthane à base de polyéther.

6. Pansement conforme à l'une des revendications précédentes,
**caractérisé en ce que**
le sac est constitué de deux surfaces membranaires appliquées l'une sur l'autre et liées l'une à l'autre sur leurs bords.

7. Pansement conforme à la revendication 6,
**caractérisé en ce qu'**
un élément d'écartement en forme de cadre est inséré entre les surfaces membranaires dans la zone de leurs bords liés entre eux.

8. Pansement conforme à l'une des revendications précédentes,
**caractérisé en ce que**
dans le sac est introduit un sac interne qui est constitué d'une paroi en mouse de polyuréthane à pores ouverts dont le diamètre est ≤ 0,4 mm et ayant une faible résistance au déchirement.
